# FASCICULE DE BREVET EUROPEEN

(11) **EP 1 511 790 B1**
(45) Date de publication et mention de la délivrance du brevet: **10.07.2013**
(21) Numéro de dépôt: 03740692.3
(22) Date de dépôt: 03.06.2003
(51) Int. Cl.: C08G 69/10, A61K 47/34, A61K 47/42, A61K 9/51, C08G 69/48

(54) **POLYAMINOACIDES FONCTIONNALISES PAR DE L'ALPHA-TOCOPHEROL ET LEURS APPLICATIONS NOTAMMENT THERAPEUTIQUES**
ALPHA-TOCOPHEROL FUNKTIONALISIERTE POLYAMINOSAÜRE UND DEREN ANWENDUNGEN, INSBESONDERE IM THERAPEUTISCHEN BEREICH
POLYAMINOACIDS FUNCTIONALIZED BY ALPHA TOCOPHEROL AND USES THEREOF, PARTICULAR FOR THERAPEUTIC APPLICATIONS

(30) Priorité: 07.06.2002 FR 0207008
(43) Date de publication de la demande: 09.03.2005
(73) Titulaire: FLAMEL TECHNOLOGIES, 69200 Vénissieux (FR)
(72) Inventeur: CHAN, You-Ping, F-69008 LYON (FR); ANGOT, Stéphanie, F-33200 Bordeaux (FR); BREYNE, Olivier, F-69004 LYON (FR)
(74) Mandataire: Cabinet Plasseraud
(86) Numéro de dépôt international: PCT/FR2003/050003
(87) Numéro de publication internationale: WO 2003/104303

(56) Documents cités:
- EP-A- 0 179 023
- WO-A-87/03891
- US-B1- 6 320 017

## Description

La présente invention concerne des nouveaux matériaux à base de polyaminoacides biodégradables, utiles notamment pour la vectorisation de principe(s) actif(s) (PA).

L'invention vise aussi de nouvelles compositions pharmaceutiques, cosmétiques, diététiques ou phytosanitaires à base de ces polyaminoacides. Ces compositions peuvent être du type de celles permettant la vectorisation de PA et se présentant de préférence sous forme d'émulsions, de micelles, de particules, de gels, d'implants ou de films.

Les PA considérés sont, avantageusement, des composés biologiquement actifs et qui peuvent être administrés à un organisme animal ou humain par voie orale, parentérale, nasale, vaginale, oculaire, sous-cutanée, intraveineuse, intra-musculaire, intradermique, intrapéritonéale, intracérébrale, buccale, etc.

Les PA plus particulièrement mais non limitativement concernés par l'invention sont des protéines, des glycoprotéines, des peptides, des polysaccharides, des lipopolysaccharides, des oligo ou des polynucléotides, et des molécules organiques. Mais il peut aussi s'agir de produits cosmétiques ou de produits phytosanitaires, tels que des herbicides, des insecticides, des fongicides, etc.

Dans le domaine de la vectorisation des principes actifs notamment médicamenteux, il existe un besoin, dans beaucoup de cas :
- de les protéger contre la dégradation (hydrolyse, précipitation sur site, digestion enzymatique etc..) jusqu'à ce qu'ils atteignent leur site d'action,
- et/ou de contrôler leur vitesse de libération afin de maintenir un niveau constant sur une durée définie, soit
- et/ou de véhiculer (en les protégeant) au site d'action.

A ces fins, plusieurs types de polymères ont été étudiés et certains sont même disponibles commercialement. On peut citer par exemple les polymères du type polylactique, polylactique-glycolique, polyoxyethylène-oxypropylène, polyaminoacide ou encore polysaccharide. Ces polymères constituent des matières premières permettant de fabriquer, par exemple, des implants massiques, des microparticules, des nanoparticules, des vésicules, des micelles ou des gels. Outre le fait que ces polymères doivent être adaptés à la fabrication de tels systèmes, ils doivent également être biocompatibles, non-toxiques, non-immunogènes, économiques et ils doivent pouvoir être facilement éliminés du corps et/ou biodégradables. Sur ce dernier aspect, il est de surcroît essentiel que la biodégradation dans l'organisme génère des produits non-toxiques.

A titre d'illustration de l'art antérieur concernant des polymères employés comme matières premières pour la réalisation de systèmes de vectorisation de PA, divers brevets ou demandes de brevet ou articles scientifiques sont évoqués ci-après.

Le brevet US 4,652,441 décrit des microcapsules de polylactide encapsulant l'hormone LH-RH. Ces microcapsules sont produites en préparant une émulsion eau-dans-huile-dans-eau et comprennent une couche interne aqueuse contenant l'hormone, une substance (gélatine) fixant cette dernière, une couche huileuse de polylactide, ainsi qu'une couche externe aqueuse (Alcool polyvinylique). La libération du PA peut se faire sur une période de plus de 2 semaines après injection sous-cutanée.

Le brevet US 6,153,193 décrit des compositions à base de micelles de poly(oxyéthylène)-poly(oxypropylène) amphiphiles, pour la vectorisation d'anti-cancéreux tel que l'adriamycine.

Akiyoshi et al. (J. Controlled Release 1998, 54, 313-320) décrivent des pullulans qui sont rendus hydrophobes par greffage de cholestérol et qui forment des nanoparticules dans l'eau. Ces nanoparticules aptes à se complexer de manière réversible avec l'insuline, forment des suspensions colloïdales stables.

Le brevet US 4,351,337 décrit des copolyaminoacides amphiphiles, à base de leucine et de glutamate, utilisables sous forme d'implants ou de microparticules pour la libération contrôlée de principes actifs. La libération de ces derniers peut se faire sur une durée très longue dépendant de la vitesse de dégradation du polymère.

Le brevet US 4,888,398 décrit des polymères à base de polyglutamate ou polyaspartate, et éventuellement polyleucine, avec des groupements pendants de type alkyloxycarbonylméthyle, placés de façon aléatoire sur la chaîne polyaminoacide. Ces polyaminoacides, greffés par des groupements latéraux e.g. méthoxycarbonylméthyle, sont utilisables sous forme d'implants biodégradables contenant un PA à libération prolongée.

Le brevet US 5,904,936 décrit des nanoparticules obtenues à partir d'un polymère bloc polyleucine-polyglutamate, aptes à former des suspensions colloïdales stables et capables de s'associer spontanément avec des protéines biologiquement actives sans les dénaturer. Ces dernières peuvent ensuite être libérées in vivo de manière contrôlée, sur une longue période.

La demande de brevet WO 00/30618 décrit des nanoparticules obtenues à partir d'un polymère bloc poly(glutamate de sodium)(polyglutamate de méthyle, éthyle, hexadécyle ou dodécyle), aptes à former des suspensions colloïdales stables et capables de s'associer spontanément avec des protéines biologiquement actives sans les dénaturer. Ces dernières peuvent ensuite être libérées in vivo de manière contrôlée, sur une longue période.

Ces copolyaminoacides amphiphiles sont modifiés par la présence d'une chaîne latérale alkyle hydrophobe.

Le brevet US 5,449,513 décrit des copolymères bloc amphiphiles comprenant un bloc polyoxyéthylène et un bloc polyaminoacide, par exemple poly(bêta-benzyl-L-aspartate). Ces polymères polyoxyéthylène-polybenzylaspartate forment des micelles qui sont aptes à encapsuler des molécules actives hydrophobes telles que l'adryamicine ou l'indométhacine.

La demande de brevet WO 99/61512 décrit des polylysines et des polyornithines fonctionnalisées par un groupe hydrophobe (acide palmitique relié à la polylysine ou ornithine et un groupe hydrophile (polyoxyéthylène). Ces polymères, par exemple la polylysine greffée avec des chaînes polyoxyéthylène et palmitoyle forment en présence de cholestérol des vésicules capables d'encapsuler la doxorubicine ou l'ADN.

Il est par ailleurs connu de recourir à des dérivés de vitamine E, et plus précisément d'alpha-tocophérol, pour construire des systèmes de vectorisation de PA.

La vitamine E naturelle est constituée d'un mélange de composés dénommés tocophérols (voir Burton et Ingold, Acc. Chem. Res. 1986, 19, 194-201) et dans ce mélange, le dérivé alpha-tocophérol est largement majoritaire. La vitamine E et certains de ses dérivés sont aujourd'hui utilisés comme source de vitamine ou comme antioxydant dans des aliments et produits cosmétiques. Pour ces utilisations courantes, on trouve la vitamine E sous sa forme D-alpha-tocophérol (sa forme naturelle) ou sous sa forme D,L-alpha-Tocophérol (forme racémique et synthétique). Ces deux produits sont considérés comme essentiellement non toxiques à des doses bien au-delà des doses thérapeutiques. La structure de l'alpha-tocophérol est la suivante.

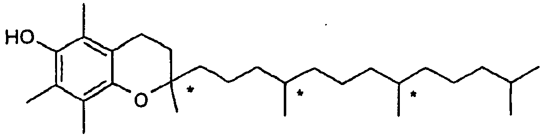

Les positions chirales sont marquées d'un astérisque. La forme naturelle possède les configurations R, R, R et la forme synthétique est un mélange où les carbones chiraux sont indépendamment R ou S.

S'agissant des dérivés de la vitamine E utilisés dans le domaine de la vectorisation de principes actifs, il n'existe, à ce jour et à la connaissance des inventeurs, aucun produit polymère à base d'alpha-tocophérol, à l'exception de polymères de type polyoxyéthylène dont une extrémité est greffée par des groupements alpha-tocophérol-succinate. Est d'ailleurs disponible sur le marché, du PolyEthylèneGlycol greffé alpha-tocophérol-succinate en bout de chaîne (vitamine E PEGylée), commercialisé sous la dénomination TPGS 1000, par la société Eastman Chemical, Ltd. Ce produit breveté en 1954 (US 2,680,749) est aujourd'hui utilisé comme source de vitamine E par voie orale. Ce polymère, de même que l'alpha-tocophérol-succinate et l'alpha-tocophérol non modifié, ont été proposés pour la vectorisation de principes actifs.

Le brevet US 5,869,703 décrit des composés voisins dans lesquels la chaîne polyoxyéthylène comporte à une extrémité de l'alpha-tocophérol et à son autre extrémité un résidu (méth)acrylique. Ces dérivés d'alpha-tocophérol sont utilisés pour préparer des vésicules amphiphiles (liposomes) stables, directement employées dans des applications cosmétiques.

La demande de brevet WO 00/71163 décrit des formulations à base de PolyEthylèneGlycol greffé alpha-tocophérol-succinate en bout de chaîne (TPGS 1000) et d'alpha-tocophérol pour la solubilisation de paclitaxel (produit anticancéreux). A ce jour, la toxicité liée à la partie polyoxyéthylène n'est pas connue et on sait que le polyoxyéthylène n'est pas dégradé in vivo. De plus, ce composé ne contient qu'un seul motif d'alpha-tocophérol par chaîne de polymère et il a des propriétés en solution assimilables à celles des tensioactifs. En tout état de cause, l'utilisation de ce produit pour la vectorisation conduirait à des associations polymère - principe actif peu stables.

Le brevet EP 0 243 446 décrit l'utilisation d'(hémi)succinate d'alpha-tocophérol (dérivé d'acide organique d'alpha-tocophérol) pour la fabrication de vésicules en combinaison avec un sel d'amine. Ces vésicules peuvent être utilisées pour l'encapsulation de divers principes actifs incluant des petites molécules, des peptides et des protéines. De manière générale, il est indiqué dans ce brevet que l'acide organique peut être un aminoacide ou un polyaminoacide. Toutefois, aucune précision n'est donnée à cet égard. Seuls des (hémi)succinates d'alpha-tocophérol sont exemplifiés.

Ainsi, même s'il existe de très nombreuses solutions techniques dans l'art antérieur, développées et proposées pour la vectorisation des principes actifs médicamenteux, la réponse à l'ensemble des exigences est difficile à obtenir et demeure insatisfaisante.

Dans ce contexte, l'un des objectifs essentiels de la présente invention est de fournir une nouvelle matière première polymère, susceptible d'être utilisée pour la vectorisation de PA et permettant de satisfaire de manière optimale à toutes les spécifications du cahier des charges :
○ biocompatibilité,
○ biodégradabilité,
○ aptitude à se transformer aisément et économiquement en particules de vectorisation de principes actifs,
○ ces particules étant elles-même propres:
   ■ à former des suspensions colloïdales aqueuses stables,
   ■ à s'associer facilement avec de nombreux principes actifs,
   ■ et à libérer ces principes actifs in vivo.

Cet objectif, parmi d'autres, est atteint par la présente invention qui concerne tout d'abord des polyaminoacides amphiphiles comprenant des unités aspartiques et/ou des unités glutamiques, caractérisés en ce qu'au moins une partie de ces unités sont porteuses de greffons comportant au moins un motif alpha-tocophérol.

Ces nouveaux polymères ont un squelette biodégradable à base de polyaminoacides porteurs de chaînes latérales comprenant de l'alpha-tocophérol. Ces polymères présentent des propriétés d'association et/ou d'encapsulation surprenantes en comparaison avec des produits analogues et de plus, ils sont facilement dégradés en présence d'enzymes.

Il est du mérite de la demanderesse d'avoir eu l'idée de combiner, de façon tout à fait judicieuse et avantageuse, des polyaminoacides particuliers polyAsp et/ou polyGlu, biodégradables avec des greffons à base d'alpha-tocophérol (vitamine E) pour la vectorisation de PA.

Au sens de l'invention le terme *"polyaminoacide"* couvre aussi bien les oligoaminoacides comprenant de 2 à 20 unités aminoacide que les polyaminoacides comprenant plus de 20 unités aminoacide.

De préférence, les polyaminoacides selon la présente invention sont des oligomères ou des homopolymères comprenant des unités récurrentes aminoacide glutamique ou aspartique ou des copolymères comprenant un mélange de ces deux types d'unités aminoacide, lesdites unités étant partiellement substituées par des greffons comportant de l'alpha-tocophérol. Les unités considérées dans ces polymères sont des acides aminés ayant la configuration D, L ou D, L et sont liées par leurs positions alpha ou gamma pour l'unité glutamate ou glutamique et alpha ou bêta pour l'unité aspartique ou aspartate.

Les unités aminoacide préférées sont celles ayant la configuration L et une liaison de type alpha.

De manière plus préférée encore, les polyaminoacides selon l'invention répondent à la formule générale (I) suivante : 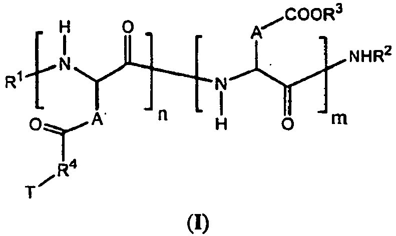 dans laquelle :
■ R¹ représente un H, un groupe acyle linéaire en C2 à C10 ou ramifié en C3 à C10, ou un pyroglutamate ;
■ R² représente un H, un alkyle linéaire en C2 à C10 ou ramifié en C3 à C10, un benzyle, une unité acide aminé terminale ;
■ R³ est un H ou une entité cationique, de préférence sélectionnée dans le groupe comprenant :
   - les cations métalliques avantageusement choisis dans le sous-groupe comprenant : le sodium, le potassium, le calcium, le magnésium,
   - les cations organiques avantageusement choisis dans le sous-groupe comprenant :
      - les cations à base d'amine,
      - les cations à base d'oligoamine,
      - les cations à base de polyamine (la polyethylèneimine étant particulièrement préférée),
      - les cations à base d'acide(s) aminé(s) avantageusement choisis dans la classe comprenant les cations à base de lysine ou d'arginine,
   - ou les polyaminoacides cationiques avantageusement choisis dans le sous-groupe comprenant la polylysine ou l'oligolysine ;
      ■ R⁴ représente une liaison directe ou un "espaceur" à base de 1 à 4 unités acide aminé ;
      ■ A représente indépendamment un radical -CH₂- (unité aspartique) ou - CH₂-CH₂- (unité glutamique) ;
      ■ n/(n+m) est défini comme le taux de greffage molaire et varie de 0,5 à 100 % molaire ;
      ■ n + m varie de 3 à 1000, de préférence entre 30 et 300 ;
      ■ T représente un motif alpha-tocophérol.

Pour ces utilisations courantes, on trouve la vitamine E sous sa forme D-alpha-tocophérol (sa forme naturelle) ou sous sa forme D,L-alpha-Tocophérol (forme racémique et synthétique). Ces deux produits sont considérés comme essentiellement non toxiques à des doses bien au-delà des doses thérapeutiques. Dans le cadre de l'invention, ces deux formes d'alpha-tocophérol sont préférées.

L'alpha-tocophérol est d'origine naturelle ou synthétique.

Selon un premier mode de réalisation de l'invention, les polyaminoacides sont des homopolymères d'alpha-L-glutamate ou d'acide alpha-L-glutamique.

Selon un deuxième mode de réalisation de l'invention, les polyaminoacides sont des homopolymères d'alpha-L-aspartate ou d'acide alpha-L-aspartique.

Selon un troisième mode de réalisation de l'invention, les polyaminoacides sont des copolymères d'alpha-L-aspartate/alpha-L-glutamate ou d'acide alpha-L-aspartique/acide alpha-L-glutamique.

Avantageusement, la distribution des unités aspartiques et/ou glutamiques porteuses de greffons comportant au moins un motif alpha-tocophérol est telle que les polymères ainsi constitués sont soit aléatoires, soit de type bloc, soit de type multibloc.

Selon un autre mode de définition, les polyaminoacides selon l'invention ont une masse molaire qui se situe entre 2 000 et 100 000 g/mole, et de préférence entre 5 000 et 40 000 g/mole.

Il est par ailleurs préférable que le taux de greffage molaire en alpha-tocophérol des polyaminoacides selon l'invention, soit compris entre 3 et 70 %, et de préférence entre 5 et 50 %.

De manière remarquable, les polyaminoacides de l'invention sont susceptibles d'être utilisés de plusieurs façons selon le taux de greffage. Les méthodes de mise en forme d'un polymère pour fencapsulation d'un principe actif sous les diverses formes visées par l'invention sont connues de l'homme de l'art. Pour plus de détails, on peut se référer, par exemple à ces quelques références particulièrement pertinentes :
*"*Microspheres, Microcapsules and Liposomes ; vol 1. Preparation and chemical applications" Ed. R. Arshady, Citus Books 1999. ISBN : 0-9532187-1-6.
*"Sustained-Release Injectable Products"* Ed.J. Senior et M. Radomsky, Interpharm Press 2000. ISBN : 1-57491-101-5.
*"*Colloidal Drug Delivery Systems" Ed. J. Kreuter, Marcel Dekker, Inc. 1994. ISBN : 0-8247-9214-9.
*"*Handbook of Pharmaceutical Controlled Release Technology" Ed. D.L. Wise, Marcel Dekker, Inc. 2000. ISBN : 0-8247-0369-3.

Les polyaminoacides sont en outre extrêmement intéressants, du fait qu'à un taux de greffage relativement faible de l'ordre de 3 à 10 %, ils forment dans l'eau à pH 7,4 (par exemple avec un tampon phosphate) des suspensions colloïdales ou gels en fonction de la concentration de polymères. De plus, les particules de polyaminoacides formant la phase dispersée de la suspension colloïdale, peuvent s'associer aisément avec des principes actifs tels que des protéines, peptides ou petites molécules. La mise en forme préférée est celle décrite dans la demande de brevet WO 00/30618 de la demanderesse et qui consiste à disperser le polymère dans l'eau et à incuber la solution en présence d'un PA. Cette solution peut ensuite être filtrée sous 0,2 µm puis directement injectée à un patient.

Au-delà de 10 % de taux de greffage, le polymère peut former des microparticules capables d'associer ou d'encapsuler des PA. Dans ce contexte, la mise en forme des microparticules peut se faire en co-solubilisant le PA et le polymère dans un solvant organique approprié puis le mélange est précipité dans l'eau. Les particules sont ensuite récupérées par filtration et peuvent ensuite être utilisées pour une administration par voie orale (sous forme de gélule, sous forme compactée et/ou enrobée ou bien encore sous forme dispersée dans une huile) ou par voie parentérale après redispersion dans l'eau.

A des taux supérieurs à 30 % de greffage, la redispersion du polymère en phase aqueuse devient plus difficile du fait de la quantité plus faible des fonctions carboxylate ionisables et le polymère précipite. Dans ce cas, le polymère peut être solubilisé dans un solvant biocompatible tel que la N-méthylpyrolidone ou une huile appropriée telle que le Migliol® puis injecté en intramusculaire ou sous-cutanée ou dans une tumeur. La diffusion du solvant ou de l'huile conduit à la précipitation du polymère sur le site d'injection et forme ainsi un dépôt. Ces dépôts assurent ensuite une libération contrôlée par diffusion et/ou par érosion et/ou par dégradation hydrolytique ou enzymatique du polymère.

De façon générale, les polymères de l'invention, sous forme neutre ou ionisée, sont utilisables seuls ou dans une composition liquide, solide ou gel et dans un milieu aqueux ou organique.

Il convient de comprendre que le polymère à base de polyaminoacides contient des fonctions carboxyliques qui sont soit neutres (forme COOH), soit ionisées selon le pH et la composition. Pour cette raison, la solubilité dans une phase aqueuse est directement fonction du taux de COOH libre (non greffé par la vitamine E) et du pH. En solution aqueuse, le contre-cation peut être un cation métallique tel que le sodium, le calcium ou le magnésium, ou un cation organique tel que la triéthanolamine, le tris(hydroxyméthyl)-aminométhane ou une polyamine telle que la polyéthylèneimine.

Les polymères de l'invention sont obtenus par des méthodes connues de l'homme de l'art. Les polyaminoacides peuvent être obtenus au moins de deux façons :
- greffage de l'alpha-tocophérol sur un polyaminoacide, ou
- polymérisation des dérivés NCA d'alpha-tocophérol suivie d'une hydrolyse sélective.

Dans le premier cas, on prépare par exemple un polyaminoacide, homopolyglutamate, homopolyaspartate ou un copolymère glutamate/aspartate, bloc, multibloc ou aléatoire selon des méthodes classiques.

Pour l'obtention de polyaminoacides de type alpha, la technique la plus courante est basée sur la polymérisation d'anhydrides de N-carboxy-aminoacides (NCA), décrite, par exemple, dans l'article *"*Biopolymers, 1976, 15, 1869 et dans l'ouvrage de H.R. Kricheldorf "alpha-Aminoacid-N-carboxy Anhydride and related Heterocycles" Springer Verlag (1987). Les dérivés d'NCA sont de préférence des dérivés NCA-O-Me, NCA-O-Et ou NCA-O-Bz (Me = méthyl, Et = Ethyle et Bz = Benzyle). Les polymères sont ensuite hydrolysés dans des conditions appropriées pour obtenir le polymère sous sa forme acide. Ces méthodes sont inspirées de la description donnée dans le brevet FR 2 801 226 de la demanderesse. Un certain nombre de polymères utilisables selon l'invention, par exemple, de type poly(acide alpha-L-aspartiqtie), poly(acide alpha-L-glutamique), poly(acide alpha-D-glutamique) et poly(acide gamma-L-glutamique) de masses variables sont disponibles commercialement. Le poly(acide aspartique) de type alpha-bêta est obtenu par condensation de l'acide aspartique (pour obtenir un polysuccinimide) suivie d'une hydrolyse basique (voir Tomida et al. Polymer 1997, 38, 4733-36).

Le couplage de l'alpha-tocophérol avec une fonction acide est réalisé aisément par réaction du polyaminoacide avec la vitamine E en présence d'un carbodiimide comme agent de couplage et de préférence, un catalyseur tel que le 4-dimethylaminopyridine et dans un solvant approprié tel que le dimethylformamide (DMF), la N-méthyl pyrrolidone (NMP) ou le dimethylsulfoxide (DMSO). Le carbodiimide est, par exemple, le dicyclohexylcarbodiimide ou le diisopropylcarbodiimide. Le taux de greffage est contrôlé chimiquement par la stoechiométrie des constituants et réactifs ou le temps de réaction.

Dans le deuxième cas, on synthétise un dérivé NCA de l'alpha-tocophérol de structure suivante. La synthèse est analogue à celle décrite pour le N-carboxyanhydride de stéaryl-glutamate par Poché et al. Macromolecules 1995, 28, 6745-53.

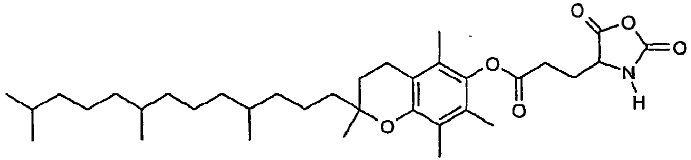

Le dérivé NCA de l'alpha-tocophérol-glutamate est ensuite copolymérisé par exemple avec le NCA de benzyl-glutamate et pour obtenir les fonctions glutamate ou glutamique, on réalise une réaction d'hydrolyse sélective des fonctions benzyles dans un mélange d'acide trifluoroacétique et d'acide bromohydrique à température ambiante. Il convient de noter que cette deuxième voie de synthèse permet de réaliser aisément des copolymères aléatoires, blocs ou multiblocs simplement en modifiant l'ordre d'ajout des monomères.

Le couplage de la vitamine E via un espaceur constitué de 1 à 4 acides aminés peut être réalisé par réactions successives de la vitamine E avec des acides aminés, protégés de façon appropriée, puis déprotégés pour avoir une fonction amine greffable sur le polymère ou par réaction avec un oligopeptide. Par exempte, la synthèse d'un alpha-tocophérol avec un motif leucine est réalisée selon une méthode générale bien connue de l'homme de l'art et selon le schéma suivant :

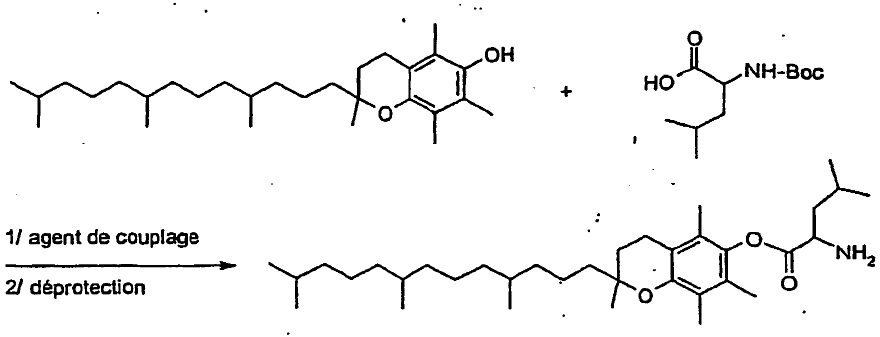

Il convient de noter que le greffage direct de l'alpha-tocophérol sur le polymère se fait via une fonction ester alors que dans le cas de la présence d'un espaceur à base d'acide(s) aminé(s), elle se fait via une fonction amide. Comme pour la réalisation d'une liaison ester, la liaison amide peut se faire de la même manière en utilisant un agent de couplage classique tel qu'un dialkyl-carbodiimide.

Selon une variante de l'invention, les polyaminoacides qu'elle concerne, sont non seulement porteurs de greffons α-tocophérol mais également, par molécule, d'au moins un greffon de type polyalkylène glycol lié à une unité glutamate ou aspartate et de préférence de formule (II) suivante : 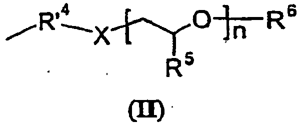 dans laquelle :
- R'⁴ représente une liaison directe ou un "espaceur" à base de 1 à 4 unités acide aminé;
- X est un hétéroatome choisi dans le groupe comportant l'oxygène, l'azote ou le soufre;
- R⁵ et R⁶ représentent indépendamment un H, un alkyle linéaire en C1 à C4;
- n varie de 3 à 1000.

De préférence, le polyalkylèneglycol est un polyethylène glycol.

Suivant une autre caractéristique préférée de l'invention, le pourcentage molaire de greffage du polyalkylène glycol varie de 1 à 30 %.

Le greffage de ces groupements latéraux pendants (Il) s'effectue de manière connue en soi et selon des techniques à la portée de l'homme du métier, par exemple par formation de liaisons amide, ester ou thioester avec les carboxyles des monomères glutamates et/ou aspartates. Ces techniques peuvent notamment être celles utilisées pour le greffage d'alpha-tocophérol sur un squelette polyaminoacide, lesdites techniques étant décrites dans la présente demande.

Selon un autre de ses aspects, l'invention vise une composition pharmaceutique, cosmétique, diététique ou phytosanitaire comprenant au moins l'un des polyaminoacides tels que définis ci-dessus.

Selon une déclinaison avantageuse de l'invention, cette composition comprend, outre l'alpha-tocophérol, au moins un principe actif, qui peut être thérapeutique, cosmétique, diététique ou phytosanitaire.

De préférence, le principe actif est une protéine, une glycoprotéine, un polysaccharide, un lipopolysaccharide, un oligonucléotide, un polynucléotide ou un peptide.

Plus préférentiellement encore, le principe actif est une "petite molécule" organique hydrophobe, hydrophile ou amphiphile.

Par "petite molécules", on désigne notamment selon le présent exposé, des molécules non-protéiniques.

Cette composition peut être sous forme de nanoparticules, de microparticules, de solutions, d'émulsions, de suspensions, de gels, de micelles, d'implants, de poudres ou de films.

Suivant l'une de ses formes particulièrement préférées, la composition, chargée ou non en principe actif(s), est une suspension colloïdale stable de nanoparticules et/ou de microparticules et/ou de micelles de polyaminoacides, dans une phase aqueuse.

La composition selon l'invention, dès lors qu'elle est pharmaceutique, peut être administrée par voie orale, parentérale, nasale, vaginale, oculaire, sous-cutanée, intraveineuse, intramusculaire, intradermique, intrapéritonéale, intracérébrale ou buccale.

Il est également envisageable que la composition soit sous forme de solution dans un solvant biocompatible, susceptible d'être injectée en sous-cutané, intramusculaire ou dans une tumeur.

Selon une autre variante, la composition selon l'invention est formulée de telle sorte qu'elle soit injectable et qu'elle soit apte à former un dépôt sur le site d'injection.

L'invention vise aussi des compositions qui comprennent des polyaminoacides selon l'invention et des principes actifs et qui sont susceptibles d'être utilisées pour la préparation :
- de médicaments, en particulier pour administration orale, nasale, vaginale, oculaire, sous-cutanée, intraveineuse, intramusculaire, intradermique, intrapéritonéale ou intracérébrale, les principes actifs de ces médicaments pouvant être, notamment, des protéines, des glycoprotéines, des protéines liées à une ou plusieurs chaînes polyalkylèneglycol {par exemple polyéthylèneglycol (PEG), on parle alors de protéines "PEGylées"}, des peptides, des polysaccharides, des lipopolysaccharides, des oligonucléotides, des polynucléotides et des petites molécules organiques hydrophobes, hydrophiles ou amphiphiles ;
- et/ou des nutriments ;
- et/ou de produits cosmétiques ou phytosanitaires.

Selon encore un autre de ses aspects, l'invention vise un procédé de préparation:
- de médicaments, en particulier pour administration orale, nasale, vaginale, oculaire, sous-cutanée, intraveineuse, intramusculaire, intradermique, intrapéritonéale ou intracérébrale, les principes actifs de ces médicaments pouvant être, notamment, des protéines, des glycoprotéines, des protéines liées à une ou plusieurs chaînes polyalkylèneglycol {par exemple polyéthylèneglycol (PEG), on parle alors de protéines "PEGylées"}, des peptides, des polysaccharides, des lipopolysaccharides, des oligonucléotides, des polynucléotides et des petites molécules organiques hydrophobes, hydrophiles ou amphiphiles ;
- et/ou des nutriments ;
- et/ou de produits cosmétiques ou phytosanitaires ;
ce procédé étant caractérisé en ce qu'il consiste essentiellement à mettre en oeuvre au moins un polyaminoacide tel que défini ci-dessus et/ou la composition elle aussi décrite supra.

Comme indiqué ci-dessus, les techniques d'association d'un ou de plusieurs PA aux polyaminoacides greffés alpha-tocophérol selon l'invention, sont décrites notamment dans la demande de brevet WO 00/30618.

L'invention concerne également une méthode de traitement thérapeutique consistant essentiellement à administrer la composition telle que décrite dans le présent exposé, par voie orale, parentérale, nasale, vaginale, oculaire, sous-cutanée, intraveineuse, intramusculaire, intradermique, intrapéritonéale, intracérébrale ou buccale.

Selon un mode particulier de mise en oeuvre, la méthode de traitement thérapeutique consiste essentiellement à utiliser une composition telle que décrite supra sous forme de solution dans un solvant biocompatible puis de l'injecter en sous-cutané, intramusculaire ou dans une tumeur, de préférence de manière à ce qu'elle forme un dépôt sur le site d'injection.

Comme exemples de PA susceptibles d'être associés aux polyaminoacides selon l'invention, qu'ils soient ou non sous forme de (nano ou micro)particules, on peut citer :
○ les protéines telles que l'insuline, les interférons, les hormones de croissance, les interleukines, l'érythropoïétine ou les cytolcines ;
○ les peptides tels que le leuprolide ou la cyclosporine ;
○ les petites molécules telles que celles appartenant à la famille des anthracyclines, des taxoïdes ou des camptothécines ;
○ et leurs mélanges.

L'invention sera mieux comprise et ses avantages et variantes de mise en oeuvre ressortiront bien des exemples qui suivent et qui décrivent la synthèse des polyaminoacides greffés alpha-tocophérol, leur transformation en système de vectorisation de PA (suspension aqueuse stable de nanoparticules) et la démonstration de la capacité d'un tel système de s'associer à des PA (petites molécules organiques, protéines...) pour former des compositions pharmaceutiques.

### Exemple 1 : Polymère P1

### Synthèse d'un polyglutamate greffé par l'apha-tocophérol d'origine synthétique

Le polymère d'alpha-L-polyglutamate, de masse équivalente à environ 10 000 par rapport à un standard en polyoxyéthylène, est obtenu par polymérisation de NCAGIuOMe, suivie d'une hydrolyse, comme décrit dans la demande de brevet FR 2 801 226. On solubilise 5,5 g de ce polymère d'alpha-L-polyglutamate dans 92 ml de diméthylformamide (DMF), en chauffant à 40°C pendant 2 heures. Une fois le polymère solubilisé, on laisse revenir la température à 25°C et on ajoute successivement 1,49 g de D,L-alpha-tocophérol (> 98 % obtenu de Fluka®) préalablement solubilisé dans 6 ml de DMF, 0,09 g de 4-dimethylaminopyridine préalablement solubilisé dans 6 ml de DMF et 0,57 g de diisopropylcarbodiimide préalablement solubilisé dans 6 ml de DMF. Après 8 heures à 25°C sous agitation, le milieu réactionnel est versé dans 800 ml d'eau contenant 15 % de chlorure de sodium et d'acide chlorhydrique (pH 2). Le polymère précipité est ensuite récupéré par filtration, lavé par de l'acide chlorhydrique 0,1 N puis par de l'eau. Le polymère est ensuite resolubilisé dans 75 ml de DMF puis reprécipité dans de l'eau contenant comme précédemment du sel et de l'acide à pH 2. Après 2 lavages à l'eau, on lave plusieurs fois par de l'éther diisopropylique. Le polymère est ensuite séché à l'étuve sous vide à 40°C. On obtient un rendement de l'ordre de 85 %.

Le taux de greffage estimé par RMN du proton est d'environ 7,8 % et une analyse par HPLC révèle un taux résiduel de tocophérol inférieur à 0,3 %.

Mw (mesuré par GPC en éluant avec la NMP) = 17 500 g/mol (en équivalent de polyméthyl méthacrylate)

### Exemples 2, 3, 4 et 5 : Synthèse de polymères P2, P3, P4 et P5

On réalise de la même façon des polymères ayant des quantités variables de tocophérol.

**Tableau 1 :**

| Polymère | Alpha-tocophérol | Taux de greffage |
|---|---|---|
| P2 | Synthétique : D,L | 5,2 % |
| P3 | Synthétique : D,L | 12,8 % |
| P4 | Synthétique D,L | 20,0 % |
| P5 | Synthétique D,L | 50,0 % |

Dans tous les cas, la quantité de tocophérol effectivement greffé a été confirmée par RMN.

### Exemple 6 : Polymère P6

### Synthèse d'un polyglutamate greffé par l'alpha-tocophérol d'origine naturelle

De façon analogue, on synthétise le polymère P6 avec 7,3 % de D-alpha-tocophérol d'origine naturelle (à 98,5 % et obtenue de la société ADM France). La masse molaire est de 17 400 (GPC NMP, éq. PMMA).

### Exemple 7 : Analyse des polymères en solution aqueuse

Les polymères sont mis en solution dans un tampon phosphate salin à pH 7,4 à des concentrations variant de 10 à 40 mg/ml et on ajuste le pH à 7,4 par ajout de soude à 0,1 N. On observe visuellement la solubilisation.

**Tableau 2 : solubilité dans l'eau saline à pH 7,4**

| Polymère | Taux de greffage | Concentration | Aspect |
|---|---|---|---|
| P1 (D,L) | 7,8 % | 10 à 30 mg/ml | Soluble et limpide |
| P3 (D,L) | 12 % | 10 mg/ml | Précipité très fin |
| P4 (D,L) | 20 % | 10 mg/ml | Précipité très fin |
| P6 (D) | 7,3 % | 10 à 30 mg/ml | Soluble et limpide |

Tampon phosphate : 0,01 M phosphate, 0,0027 M KCI et 0,137M NaCl.

Une observation en transmission électronique des solutions limpides du polymère P1 déposé sur un support montre l'existence de nanoparticules de 15 à 25 nm. Une analyse comparative des solutions du polymère P1, P6 et l'alpha-tocophérol succinate à 15 mg/ml dans l'eau à pH 7,4 (tampon phosphate) révèle que seul l'alpha-tocophérol succinate développe une solution laiteuse caractéristiques des vésicules comme décrit dans le brevet EP 0 243 446.

### Exemple 8 : Adsorption d'un colorant sur le polymère P1

Selon l'un des objets de l'invention, les polymères peuvent être utilisés sous forme de suspension colloïdale dans l'eau et associés à un principe actif. Pour cette application, nous démontrons dans l'expérience ci-après qu'avec certains polymères, notamment ceux avec un taux de greffage de l'ordre de 5 à 10 % de tocophérol, la capacité d'adsorption est supérieure à celle d'un composé analogue de l'art antérieur.

Pour cette étude, nous avons comparé le polymère P1 avec un polymère analogue ayant une chaîne dodécanol greffé sur un polyglutamate. Ce polymère est décrit dans le brevet WO 00 30618.

L'étude est réalisée de la façon suivante : on solubilise les polymères dans une solution aqueuse à pH 7 (tampon phosphate) et on ajoute 5 mg du colorant dénommé Orange OT (Rn CAS : 2646-17-5). On laisse les solutions dans un bain d'ultrason pendant une heure pour réaliser l'association. Les solutions sont ensuite centrifugées pour éliminer le colorant non-associé et on mesure la densité optique au λmax du colorant qui se situe à 495 nm.

**Tableau 3 :**

| Polymère | Taux de greffage | Concentration polymère | DO normalisée |
|---|---|---|---|
| P1 (alpha-tocophérol) | 7,8 % molaire | 13,8 mg/ml | 1 |
| Polymère comparatif* (dodécanol) | 15 % molaire | 17,3 mg/ml | 0,45 |

| | | | |
|---|---|---|---|
| *WO 00 30618 | | | |

On constate qu'à un taux de greffage molaire inférieur de moitié et à une concentration massique en polymère un peu plus faible, le polymère P1 présente une capacité d'association du colorant Orange OT bien supérieure.

### Exemple 9 : synthèse du polymère P7

### Synthèse d'un polyglutamate ayant un greffon d'alpha-tocophérol leucine.

On synthétise d'abord le dérivé alpha-tocophérol leucine de la façon suivante.

On fait réagir le D,L-alpha-tocophérol (4,3 g) avec la BOC-Leucine (2,3 g) dans 15 ml de dichlorométhane en présence de 4-diméthylaminopyridine (244 mg) et de diisopropylcarbodiimide (1,5 g). Après 2 heures à 30°C, le produit est purifié par filtration sur une colonne de silice. On obtient 5 g du produit alpha-tocophérol leucine BOC (rendement 77 %). Sa structure est confirmée par spectroscopie RMN. La déprotection du produit est réalisée dans l'acide trifluoroacétique à une température comprise entre 5 et 10°C pendant une heure. Après purification par filtration sur silice, on isole 3,3 g du produit souhaité (rendement 78%), Sa structure est confirmée par spectroscopie RMN.

On réalise ensuite la réaction de greffage sur un acide polyglutamique dans les mêmes conditions que dans l'exemple 1, avec un taux de greffage de 7 %. La structure du polymère et le taux de greffage ont été confirmés par spectroscopie RMN.

### Exemple 10 : synthèse du polymère P8

### Synthèse d'un polyglutamate ayant un greffon d'alpha-tocopherol et un greffon de polyoxyethylène glycol.

On réalise comme dans l'exemple 1, une réaction de greffage avec 11 % molaire d'alpha-tocophérol et 2 % molaire d'un methoxypolyethylène glycol aminé de formule MeO(CH₂CH₂O)ₙCH₂CH₂NH₂ et de masse molaire 3000 (produit obtenu de la société Shearwaters). Le polymère sous sa forme acide est obtenu avec un rendement de 72 %. La RMN du proton confirme un taux de greffage d'alpha-tocophérol de 10,9 % et de polyethylène glycol de 1,9 %.

### Exemple 11 : Adsorption de l'insuline

On prépare une solution à 1 mg du polymère P1 et 7 mg d'insuline à pH 7,0 dans 1 ml d'eau et on laisse incuber pendant 2 heures. La suspension est ensuite ultrafiltrée (10000G, 20 minutes avec un seuil de 100 KDa). On dose l'insuline libre dans le filtrat par HPLC et on en déduit par différence la quantité d'insuline associée. On mesure un taux d'association qui est supérieur à 95 % par rapport à l'insuline engagée. Dans les mêmes conditions, le polymère comparatif de l'exemple 8 permet d'associer 40 %. La capacité d'adsorption du polymère P1 est donc supérieure.

### Exemple 12 : Dégradation in-vitro du polymère P1 en présence d'enzymes

On solubilise le polymère P1 à pH 7,5 (tampon phosphate et 10 mM en cation calcium) et à une concentration de 20mg/ml. On ajoute 0,1 mL de protéase (solution de 10mg/ml) et on suit la dégradation par la GPC aqueuse.

On constate une dégradation relativement rapide avec un temps de demi-vie du polymère initiale à environ 100 minutes.

## Revendications

1. Polyaminoacides comprenant des unités aspartiques et/ou des unités glutamiques, **caractérisés en ce qu'**au moins une partie de ces unités sont porteuses de greffons comportant au moins un motif alpha-tocophérol.

2. Polyaminoacides selon la revendication 1, **caractérisés par** la formule générale (I) suivante : 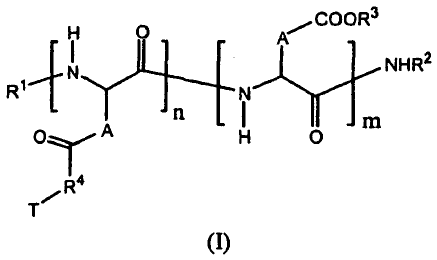 dans laquelle :
■ R¹ représente un H, un groupe acyle linéaire en C2 à C 10 ou ramifié en C3 à C10 ou un pyroglutamate ;
■ R² représente un H, un alkyle linéaire en C2 à C10 ou ramifié en C3 à C10, un benzyle, une unité acide aminé terminale ;
■ R³ est un H ou une entité cationique, de préférence sélectionnée dans le groupe comprenant :
- les cations métalliques avantageusement choisis dans le sous-groupe comprenant : le sodium, le potassium, le calcium, le magnésium,
- les cations organiques avantageusement choisis dans le sous-groupe comprenant :
• les cations à base d'amine,
• les cations à base d'oligoamine,
• les cations à base de polyamine (la polyethylèneimine étant particulièrement préférée),
• les cations à base d'acide(s) aminé(s) avantageusement choisis dans la classe comprenant les cations à base de lysine ou d'arginine,
- ou les polyaminoacides cationiques avantageusement choisis dans le sous-groupe comprenant la polylysine ou l'oligolysine ;
■ R⁴ représente une liaison directe ou un "espaceur" à base de 1 à 4 unités acide aminé ;
■ A représente indépendamment un radical -CH₂- (unité aspartique) ou - CH₂-CH₂- (unité glutamique) ;
■ n/(n+m) est défini comme le taux de greffage molaire et varie de 0,5 à 100 % molaire ;
■ n + m varie de 3 à 1000,de préférence entre 30 et 300 ;
■ T représente un motif alpha-tocophérol.

3. Polyaminoacides selon la revendication 1 ou 2, **caractérisés en ce que** l'alpha-tocophérol est d'origine naturelle.

4. Polyaminoacides selon la revendication 1 ou 2, **caractérisés en ce que** l'alpha-tocophérol est d'origine synthétique.

5. Polyaminoacides selon la revendication 2, **caractérisés en ce qu'**ils sont constitués d'un homopolymère d'alpha-L-glutamate ou d'acide alpha-L-glutamique.

6. Polyaminoacides selon la revendication 2, **caractérisés en ce qu'**ils sont constitués d'un homopolymère d'alpha-L-aspartate ou d'acide alpha-L-aspartique.

7. Polyaminoacides selon la revendication 2, **caractérisés en ce qu'**ils sont constitués d'un copolymère d'alpha-L-aspartate/alpha-L-glutamate ou d'acide alpha-L-aspartique/acide alpha-L-glutamique.

8. Polyaminoacides selon l'une quelconque des revendications 1 à 7, **caractérisés en ce que** la distribution des unités aspartiques et/ou glutamiques porteuses de greffons comportant au moins un motif alpha-tocophérol est telle que les polymères ainsi constitués sont soit aléatoires, soit de type bloc, soit de type multibloc.

9. Polyaminoacides selon l'une quelconque des revendications 1 à 8, **caractérisés en ce que** leur masse molaire se situe entre 2 000 et 100 000 g/mole, et de préférence entre 5 000 et 40 000 g/mole.

10. Polyaminoacides selon l'une quelconque des revendications 1 à 9, **caractérisés en ce que** le taux de greffage molaire se situe entre 3 et 70 %, et de préférence entre 5 et 50 %.

11. Polyaminoacides selon l'une quelconque des revendications 1 à 10, **caractérisés en ce qu'**ils sont porteurs d'au moins un greffon de type polyalkylène glycol lié à une unité glutamate ou aspartate.

12. Polyaminoacides selon la revendication 11, dans lesquels le greffon de type polyalkylène glycol lié à une unité glutamate ou aspartate a la formule (II) suivante : 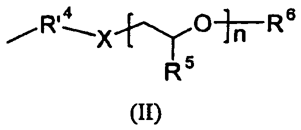 dans laquelle :
- R'⁴ représente une liaison directe ou un "espaceur" à base de 1 à 4 unités acide aminé;
- X est un hétéroatome choisi dans le groupe comportant l'oxygène, l'azote ou le soufre;
- R⁵ et R⁶ représentent indépendamment un H, un alkyle linéaire en C1 à C4;
- n varie de 3 à 1000.

13. Polyaminoacides selon la revendication 11 ou 12, **caractérisés en ce que** le polyalkylèneglycol est un polyethylène glycol.

14. Polyaminoacides selon l'une quelconque des revendications 11 à 13, **caractérisés en ce que** le pourcentage molaire de greffage du polyalkylène glycol varie de 1 à30%.

15. Composition pharmaceutique, cosmétique, diététique ou phytosanitaire comprenant au moins l'un des polyaminoacides selon l'une quelconque des revendications 1 à 14.

16. Composition selon la revendication 15, **caractérisée en ce qu'**elle comprend au moins un principe actif.

17. Composition selon la revendication 16, **caractérisée en ce que** le principe actif est une protéine, une glycoprotéine, un polysaccharide, un lipopolysaccharide, un oligonucléotide, un polynucléotide ou un peptide.

18. Composition selon la revendication 16, **caractérisée en ce que** le principe actif est une molécule non protéinique organique hydrophobe, hydrophile ou amphiphile.

19. Composition selon l'une quelconque des revendications 15 à 18, **caractérisée en ce qu'**elle peut être administrée par voie orale, parentérale, nasale, vaginale, oculaire, sous-cutanée, intraveineuse, intramusculaire, intradermique, intra péritonéale, intracérébrale ou buccale.

20. Composition selon l'une quelconque des revendications 15 à 19, **caractérisée en ce qu'**elle est sous forme d'un gel, d'une émulsion, d'une solution, d'une suspension, de micelles, de nanoparticules, de microparticules, d'une poudre ou d'un film.

21. Composition selon l'une quelconque des revendications 15 à 20, **caractérisée en ce qu'**elle est une suspension colloïdale de nanoparticules et/ou de microparticules et/ou de micelles de polyaminoacides, dans une phase aqueuse.

22. Composition selon l'une quelconque des revendications 15 à 19, **caractérisée en ce qu'**elle est sous forme de solution dans un solvant biocompatible et **en ce qu'**elle peut être injectée en sous-cutané, intramusculaire ou dans une tumeur.

23. Composition selon l'une quelconque des revendications 15 à 22, **caractérisée en ce qu'**elle est injectable et **en ce qu'**elle est apte à former un dépôt sur le site d'injection.

24. Composition selon l'une quelconque des revendications 15 à 23, **caractérisée en ce qu'**elle est destinée à la préparation :
• de médicaments, en particulier pour administration orale, nasale, vaginale, oculaire, sous-cutanée, intraveineuse, intramusculaire, intradermique, intrapéritonéale ou intracérébrale, les principes actifs de ces médicaments pouvant être, notamment, des protéines, des glycoprotéines, des protéines liées à une ou plusieurs chaînes polyalkylèneglycol {par exemple PolyEthylèneGlycol (PEG), on parle alors de protéines "PEGylées"}, des peptides, des polysaccharides, des lipopolysaccharides, des oligonucléotides, des polynucléotides et des molécules non protéiniques organiques hydrophobes, hydrophiles ou amphiphiles ;
• et/ou des nutriments ;
• et/ou de produits cosmétiques ou phytosanitaires.

25. Procédé de préparation d'un polyaminoacide tel que défini dans l'une des revendications 1 à 14,
**caractérisé en ce que** ledit polyaminoacide est obtenu par polymérisation d'anhydrides de N-carboxy-aminoacides (NCA), le polymère ainsi obtenu étant ensuite hydrolysé dans des conditions appropriées pour obtenir le polymère sous sa forme acide, ledit procédé comprenant ensuite le couplage avec un alpha-tocophérol.

26. Procédé selon la revendication 25,
**caractérisé en ce que** le couplage avec un alpha-tocophérol consiste en un couplage de l'alpha-tocophérol avec une fonction acide du polyaminoacide, ledit couplage étant réalisé par réaction, dans un solvant approprié tel que le diméthylformamide (DMF), la N-méthyl pyrrolidone (NMP) ou le diméthylsulfoxide (DMSO), du polyaminoacide avec la vitamme E en présence d'un carbodiimide comme agent de couplage.

27. Procédé de préparation d'un polyaminoacide tel que défini dans l'une des revendications 1 à 14, comprenant la synthèse dudit polyaminoacide par polymérisation d'un dérivé NCA de l'alpha-tocophérol, laquelle consiste à :
1) synthétiser un dérivé NCA de l'alpha-tocophérol-glutamate par couplage d'un alpha-tocophérol avec une fonction acide d'un N-carboxyanhydride de glutamate,
2) copolymériser ledit dérivé NCA de l'alpha-tocophérol-glutamate avec un NCA de benzyl-glutamate,
3) réaliser une réaction d'hydrolyse sélective des fonctions benzyle du copolymère, dans un mélange d'acide trifluoroacétique et d'acide bromohydrique à température ambiante.

28. Procédé selon la revendication 25,
**caractérisé en ce que** le couplage avec l'alpha-tocophérol est réalisé via un espaceur constitué de 1 à 4 acides aminés, ledit couplage étant réalisé en présence d'un agent de couplage tel qu'un dialkyl-carbodiimide et consistant en :
1) des réactions successives de la vitamine E avec des acides aminés protégés, lesquels sont ensuite déprotégés pour avoir une fonction amine greffable sur le polymère, ou,
1') une réaction de la vitamine E avec un oligopeptide,
2) un couplage de l'alpha-tocophérol-espaceur portant une fonction amine, avec la fonction acide carboxylique d'un polymère pour former une liaison amide.

29. Procédé de préparation d'une composition telle que définie dans l'une des revendications 16 à 24,
**caractérisé en ce que** le principe actif est mis en contact avec lesdits polyaminoacides tels que définis dans l'une des revendications 1 à 14.

30. Procédé de préparation :
• de médicaments, en particulier pour administration orale, nasale, vaginale, oculaire, sous-cutanée, intraveineuse, intramusculaire, intradermique, intrapéritonéale ou intracérébrale, les principes actifs de ces médicaments pouvant être, notamment, des protéines, des glycoprotéines, des protéines liées à une ou plusieurs chaînes polyalkylèneglycol {par exemple PolyEthylèneGlycol (PEG), on parle alors de protéines "PEGylées"}, des peptides, des polysaccharides, des lipopolysaccharides, des oligonucléotides, des polynucléotides et des molécules non protéiniques organiques hydrophobes, hydrophiles ou amphiphiles ;
• et/ou des nutriments ;
• et/ou de produits cosmétiques ou phytosanitaires ;
**caractérisé en ce qu'**il consiste essentiellement à mettre en oeuvre au moins un polyaminoacide selon l'une quelconque des revendications 1 à 14 et/ou la composition selon l'une quelconque des revendications 15 à 24.

## Patentansprüche

1. Polyaminosäuren mit Asparaginsäure- und/oder Glutaminsäure-Einheiten, **dadurch gekennzeichnet, dass** mindestens ein Teil dieser Einheiten Pfropfanteile mit mindestens einer alpha-Tocopherol-Einheit tragen.

2. Polyaminosäuren nach Anspruch 1, **gekennzeichnet durch** die folgende allgemeine Formel (I): 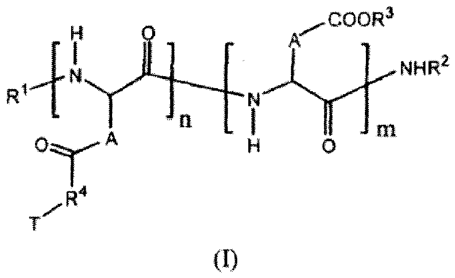 worin:
■ R¹ für H, eine lineare C₂- bis C₁₀- oder verzweigte C₃-C₁₀-Acylgruppe oder ein Pyroglutamat steht;
■ R² für H, lineares C₂- bis C₁₀- oder verzweigtes C₃-C₁₀-Alkyl, ein Benzyl oder eine endständige Aminosäure-Einheit steht;
■ R³ für H oder eine kationische Einheit steht, die vorzugsweise aus der folgenden Gruppe ausgewählt ist:
- Metallkationen, die vorteilhafterweise aus der Natrium, Kalium, Calcium und Magnesium umfassenden Untergruppe ausgewählt sind,
- organischen Kationen, die vorteilhafterweise aus der folgenden Untergruppe ausgewählt sind:
• Kationen auf Aminbasis,
• Kationen auf Oligoaminbasis,
• Kationen auf Polyaminbasis (wobei Polyethylenimin besonders bevorzugt ist),
• Kationen auf Basis einer oder mehrerer Aminosäuren, die vorteilhafterweise aus der Klasse umfassend Kationen auf Basis von Lysin oder Arginin ausgewählt sind,
- oder kationischen Polyaminosäuren, die vorteilhafterweise aus der Polylysin und Oligolysin umfassenden Untergruppe ausgewählt sind;
■ R⁴ für eine direkte Bindung oder einen "Spacer" auf Basis von 1 bis 4 Aminosäure-Einheiten steht;
■ A unabhängig für einen -CH₂-Rest (Asparaginsäure-Einheit) oder -CH₂-CH₂-Rest (Glutaminsäure-Einheit) steht;
■ n/(n+m) als molarer Pfropfgrad definiert ist und von 0,5 bis 100 Mol-% variiert;
■ n + m von 3 bis 1000 und vorzugsweise zwischen 30 und 300 variiert;
■ T für eine alpha-Tocopherol-Einheit steht.

3. Polyaminosäuren nach Anspruch 1 oder 2, **dadurch gekennzeichnet, dass** das alpha-Tocopherol natürlichen Ursprungs ist.

4. Polyaminosäuren nach Anspruch 1 oder 2, **dadurch gekennzeichnet, dass** das alpha-Tocopherol synthetischen Ursprungs ist.

5. Polyaminosäuren nach Anspruch 2, **dadurch gekennzeichnet, dass** sie aus einem alpha-L-Glutamat- oder alpha-L-Gutaminsäure-Homopolymer bestehen.

6. Polyaminosäuren nach Anspruch 2, **dadurch gekennzeichnet, dass** sie aus einem alpha-L-Aspartat- oder alpha-L-Asparaginsäure-Homopolymer bestehen.

7. Polyaminosäuren nach Anspruch 2, **dadurch gekennzeichnet, dass** sie aus einem alpha-L-Aspartat/alpha-L-Glutamat- oder alpha-L-Asparaginsäure/alpha-L-Glutaminsäure-Copolymer bestehen.

8. Polyaminosäuren nach einem der Ansprüche 1 bis 7, **dadurch gekennzeichnet, dass** Pfropfanteile mit mindestens eine alpha-Tocopherol-Einheit tragende Asparaginsäure- und/oder Glutaminsäure-Einheiten so verteilt sind, dass die so gebildeten Polymere statistisch, blockartig oder multiblockartig aufgebaut sind.

9. Polyaminosäuren nach einem der Ansprüche 1 bis 8, **dadurch gekennzeichnet, dass** ihre Molmasse zwischen 2000 und 100.000 g/mol und vorzugsweise zwischen 5000 und 40.000 g/mol liegt.

10. Polyaminosäuren nach einem der Ansprüche 1 bis 9, **dadurch gekennzeichnet, dass** ihr molarer Pfropfgrad zwischen 3 und 70% und vorzugsweise zwischen 5 und 50% liegt.

11. Polyaminosäuren nach einem der Ansprüche 1 bis 10, **dadurch gekennzeichnet, dass** sie mindestens einen Pfropfanteil vom Polyalkylenglykol-Typ, der an eine Glutamat- oder Aspartat-Einheit gebunden ist, tragen.

12. Polyaminosäuren nach Anspruch 11, in denen der Pfropfanteil vom Polyalkylenglykol-Typ, der an eine Glutamat- oder Aspartat-Einheit gebunden ist, die folgende Formel (II) aufweist: 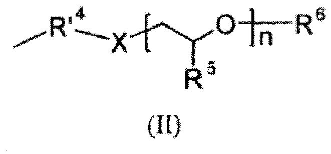 worin:
- R'⁴ für eine direkte Bindung oder einen "Spacer" auf Basis von 1 bis 4 Aminosäure-Einheiten steht;
- X für ein Heteroatom aus der Gruppe umfassend Sauerstoff, Stickstoff oder Schwefel steht;
- R⁵ und R⁶ unabhängig für H oder eine lineare C₁- bis C₄-Alkylgruppe stehen;
- n von 3 bis 1000 variiert.

13. Polyaminosäuren nach Anspruch 11 oder 12, **dadurch gekennzeichnet, dass** es sich bei dem Polyalkylenglykol um ein Polyethylenglykol handelt.

14. Polyaminosäuren nach einem der Ansprüche 11 bis 13, **dadurch gekennzeichnet, dass** der molare Pfropfgrad des Polyalkylenglykols von 1 bis 30% variiert.

15. Pharmazeutische, kosmetische, diätetische oder phytosanitäre Zusammensetzung, die mindestens eine der Polyaminosäuren nach einem der Ansprüche 1 bis 14 umfasst.

16. Zusammensetzung nach Anspruch 15, **dadurch gekennzeichnet, dass** sie mindestens einen Wirkstoff umfasst.

17. Zusammensetzung nach Anspruch 16, **dadurch gekennzeichnet, dass** es sich bei dem wirkstoff um ein Protein, ein Glykoprotein, ein Polysaccharid, ein Lipopolysaccharid, ein Oligonucleotid, ein Polynucleotid oder ein Peptid handelt.

18. Zusammensetzung nach Anspruch 16, **dadurch gekennzeichnet, dass** es sich bei dem Wirkstoff um ein hydrophobes, hydrophiles oder amphiphiles organisches Nichtproteinmolekül handelt.

19. Zusammensetzung nach einem der Ansprüche 15 bis 18, **dadurch gekennzeichnet, dass** sie auf oralem, parenteralem, nasalem, vaginalem, okulärem, subkutanem, intravenösem, intramuskulärem, intradermalem, intraperitonealem, intrazerebralem oder bukkalem Weg verabreicht werden kann.

20. Zusammensetzung nach einem der Ansprüche 15 bis 19, **dadurch gekennzeichnet, dass** sie in Form eines Gels, einer Emulsion, einer Lösung oder einer Suspension, in Form von Micellen, Nanopartikeln oder Mikropartikeln, in Form eines Pulvers oder eines Films vorliegt.

21. Zusammensetzung nach einem der Ansprüche 15 bis 20, **dadurch gekennzeichnet, dass** es sich um eine kolloidale Suspension von Nanopartikeln und/oder Mikropartikeln und/oder Micellen von Polyaminosäuren in einer wässrigen Phase handelt.

22. Zusammensetzung nach einem der Ansprüche 15 bis 19, **dadurch gekennzeichnet, dass** sie in Form einer Lösung in einem biokompatiblen Lösungsmittel und auf subkutanem oder intramuskulärem Wege oder in einen Tumor injiziert werden kann.

23. Zusammensetzung nach einem der Ansprüche 15 bis 22, **dadurch gekennzeichnet, dass** sie injizierbar ist und am Injektionsort ein Depot bilden kann.

24. Zusammensetzung nach einem der Ansprüche 15 bis 23, **dadurch gekennzeichnet, dass** sie zur Herstellung von:
• Arzneimitteln, insbesondere zur oralen, nasalen, vaginalen, okulären, subkutanen, intravenösen, intramuskulären, intradermalen, intraperitonealen oder intrazerebralen Verabreichung, wobei es sich bei den Wirkstoffen dieser Arzneimittel insbesondere um Proteine, Glykoproteine, an eine oder mehrere Polyalkylenglykolketten {beispielsweise Polyethylenglykolketten (PEG-Ketten), wobei man dann von "PEGylierten" Proteinen spricht} gebundene Proteine, Peptide, Polysaccharide, Lipopolysaccharide, Oligonucleotide, Polynucleotide und hydrophobe, hydrophile oder amphiphile organische Nichtproteinmoleküle handeln kann;
• und/oder Nährstoffen
• und/oder kosmetischen oder phytosanitären Produkten
vorgesehen ist.

25. Verfahren zur Herstellung einer Polyaminosäure gemäß einem der Ansprüche 1 bis 14,
**dadurch gekennzeichnet, dass** die Polyaminosäure durch Polymerisation von N-Carboxyaminosäureanhydriden (NCA) erhalten wird, wobei das so erhaltene Polymer dann unter zum Erhalt des Polymers in seiner Säureform geeigneten Bedingungen hydrolysiert wird, wobei das Verfahren anschließend die Kupplung mit einem alpha-Tocopherol umfasst.

26. Verfahren nach Anspruch 25,
**dadurch gekennzeichnet, dass** die Kupplung mit dem alpha-Tocopherol aus einer Kupplung des alpha-Tocopherols mit einer Säurefunktion der Polyaminosäure besteht, wobei die Kupplung durch Umsetzung der Polyaminosäure mit Vitamin E in Gegenwart eines Carbodiimids als Kupplungsmittel in einem geeigneten Lösungsmittel wie Dimethylformamid (DMF), N-Methylpyrrolidon (NMP) oder Dimethylsulfoxid (DMSO) durchgeführt wird.

27. Verfahren zur Herstellung einer Polyaminosäure gemäß einem der Ansprüche 1 bis 14, umfassend die Synthese der Polyaminosäure durch Polymerisation eines NCA-Derivats von alpha-Tocopherol, bestehend aus:
1) Synthese eines NCA-Derivats von alpha-Tocopherol-glutamat durch Kupplung eines alpha-Tocopherols mit einer Säurefunktion eines Glutamat-N-carboxyanhydrids,
2) Copolymerisation des NCA-Derivats von alpha-Tocopherol-glutamat mit einem Benzylglutamat-NCA,
3) Durchführung einer selektiven Hydrolysereaktion der Benzylfunktionen des Copolymers in einer Mischung von Trifluoressigsäure und Bromwasserstoffsäure bei Umgebungstemperatur.

28. Verfahren nach Anspruch 25,
**dadurch gekennzeichnet, dass** die Kupplung mit dem alpha-Tocopherol über einen Spacer aus 1 bis 4 Aminosäuren erfolgt, wobei die Kupplung in Gegenwart eines Kupplungsmittels wie eines Dialkylcarbodiimids durchgeführt wird und aus Folgendem besteht:
1) aufeinanderfolgenden Umsetzungen von Vitamin E mit geschützten Aminosäuren, die dann zum Erhalt einer Aminfunktion, die auf das Polymer gepfropft werden kann, entschützt werden können, oder
1') Umsetzung von Vitamin E mit einem Oligopeptid, 2) Kupplung des eine Aminfunktion tragenden alpha-Tocopherol-Spacers mit der Carbonsäurefunktion eines Polymers zur Bildung einer Amidbindung.

29. Verfahren zur Herstellung einer Zusammensetzung gemäß einem der Ansprüche 16 bis 24,
**dadurch gekennzeichnet, dass** man den Wirkstoff mit den Polyaminosäuren gemäß einem der Ansprüche 1 bis 14 in Berührung bringt.

30. Verfahren zur Herstellung von:
• Arzneimitteln, insbesondere zur oralen, nasalen, vaginalen, okulären, subkutanen, intravenösen, intramuskulären, intradermalen, intraperitonealen oder intrazerebralen Verabreichung, wobei es sich bei den wirkstoffen dieser Arzneimittel insbesondere um Proteine, Glykoproteine, an eine oder mehrere Polyalkylenglykolketten {beispielsweise Polyethylenglykolketten (PEG-Ketten), wobei man dann von "PEGylierten" Proteinen spricht} gebundene Proteine, Peptide, Polysaccharide, Lipopolysaccharide, Oligonucleotide, Polynucleotide und hydrophobe, hydrophile oder amphiphile organische Nichtproteinmoleküle handeln kann;
• und/oder Nährstoffen
• und/oder kosmetischen oder phytosanitären Produkten;
**dadurch gekennzeichnet, dass** es im Wesentlichen aus der Verwendung mindestens einer Polyaminosäure nach einem der Ansprüche 1 bis 14 und/oder der Zusammensetzung nach einem der Ansprüche 15 bis 24 besteht.

## Claims

1. Polyamino acids comprising aspartic units and/or glutamic units, **characterized in that** at least some of these units bear grafts comprising at least one alpha-tocopherol unit.

2. Polyamino acids according to claim 1, **characterized by** the general formula (I) below: 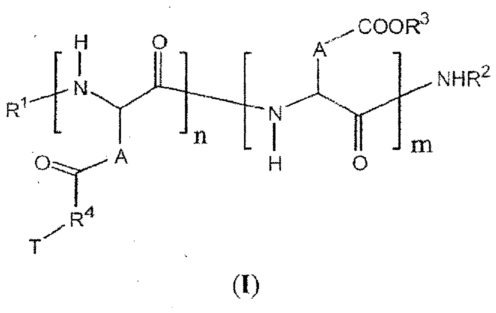 in which:
■ R¹ represents an H, a linear C2 to C10 or branched C3 to C10 acyl group or a pyroglutamate;
■ R² represents H, a C2 to C10 linear or C3 to C10 branched alkyl, a benzyl or a terminal amino acid unit;
■ R³ is an H or a cationic species preferably selected from the group comprising:
- metallic cations advantageously chosen from the subgroup comprising: sodium, potassium, calcium, magnesium,
- organic cations advantageously chosen from the subgroup comprising:
• amine-based cations,
• oligoamine-based cations,
• cations based on polyamine (polyethyleneimine being particularly preferred),
• cations based on amino acid(s) advantageously chosen from the class comprising cations based on lysine or arginine,
- or cationic polyamino acids advantageously chosen from the subgroup comprising polylysine or oligolysine;
■ R⁴ represents a direct bond or a "spacer" based on 1 to 4 amino acid units;
■ A independently represents a -CH₂- (aspartic unit) or -CH₂-CH₂- (glutamic unit) radical;
■ n/ (n+m) is defined as the molar grafting rate and ranges from 0.5 to 100 mol%;
■ n+m ranges from 3 to 1000 and preferably between 30 and 300;
■ T represents an alpha-tocopherol unit.

3. Polyamino acids according to claim 1 or 2, **characterized in that** the alpha-tocopherol is of natural origin.

4. Polyamino acids according to claim 1 or 2, **characterized in that** the alpha-tocopherol is of synthetic origin.

5. Polyamino acids according to claim 2, **characterized in that** they consist of an alpha-L-glutamate or alpha-L-glutamic acid homopolymer.

6. Polyamino acids according to claim 2, **characterized in that** they consist of an alpha-L-aspartate or alpha-L-aspartic acid homopolymer.

7. Polyamino acids according to claim 2, **characterized in that** they consist of an alpha-L-aspartate/alpha-L-glutamate or alpha-L-aspartic acid /alpha-L-glutamic acid copolymer.

8. Polyamino acids according to any one of claims 1 to 7, **characterized in that** the distribution of the aspartic and/or glutamic units bearing grafts comprising at least one alpha-tocopherol unit is such that the polymers thus composed are either random, or of block type, or of multiblock type.

9. Polyamino acids according to any one of claims 1 to 8, **characterized in that** their molar mass is between 2 000 and 100 000 g/mol, and preferably between 5 000 and 40 000 g/mol.

10. Polyamino acids according to any one of claims 1 to 9, **characterized in that** the molar grafting rate is between 3% and 70% and preferably between 5% and 50%.

11. Polyamino acids according to any one of claims 1 to 10, **characterized in that** it bears at least one graft of polyalkylene glycol type linked to a glutamate or aspartate unit.

12. Polyamino acids according to claim 11, wherein the graft of polyalkylene glycol type linked to a glutamate or aspartate unit has the formula (II) below: 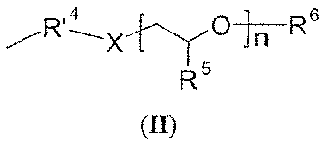 in which:
- R'⁴ represents a direct bond or a "spacer" based on 1 to 4 amino acid units;
- X is a hetero atom chosen from the group comprising oxygen, nitrogen and sulfur;
- R⁵ and R⁶ independently represent H or a linear C1 to C4 alkyl;
- n ranges from 3 to 1000.

13. Polyamino acids according to claim 11 or 12, **characterized in that** the polyalkylene glycol is a polyethylene glycol.

14. Polyamino acids according to any one of claims 11 to 13, **characterized in that** the molar grafting percentage of the polyalkylene glycol ranges from 1% to 30%.

15. Pharmaceutical, cosmetic, dietetic or plant-protection composition comprising at least one of the polyamino acids as claimed in any one of claims 1 to 14.

16. Composition according to claim 15, **characterized in that** it comprises at least one active principle.

17. Composition according to claim 16, **characterized in that** the active principle is a protein, a glycoprotein, a polysaccharide, a lipopolysaccharide, an oligonucleotide, a polynucleotide or a peptide.

18. Composition according to claim 16, **characterized in that** the active principle is a hydrophobic, hydrophilic or amphiphilic organic nonprotein molecule.

19. Composition according to any one of claims 15 to 18, **characterized in that** it may be administered via the oral, parenteral, nasal, vaginal, ocular, subcutaneous, intravenous, intramuscular, intradermal, intraperitoneal, intracerebral or buccal route.

20. Composition according to any one of claims 15 to 19, **characterized in that** it is in the form of a gel, an emulsion, a solution, a suspension, micelles, nanoparticles, microparticles, a powder or a film.

21. Composition according to any one of claims 15 to 20, **characterized in that** it is a colloidal suspension of nanoparticles and/or microparticles and/or micelles of polyamino acids, in an aqueous phase.

22. Composition according to any one of claims 15 to 19, **characterized in that** it is in the form of a solution in a biocompatible solvent and **in that** it can be injected subcutaneously, intramuscularly or into a tumor.

23. Composition according to any one of claims 15 to 22, **characterized in that** it is injectable and **in that** it is capable of forming a deposit at the injection site.

24. Composition according to any one of claims 15 to 23, **characterized in that** it is for the preparation:
• of medicinal products, in particular for oral, nasal, vaginal, ocular, subcutaneous, intravenous, intramuscular, intradermal, intraperitoneal or intracerebral administration, the active principles of these medicinal products possibly being, especially, proteins, glycoproteins, proteins linked to one or more polyalkylene glycol chains {for example polyethylene glycol (PEG), in which case they are referred to as "PEGylated" proteins}, peptides, polysaccharides, lipopolysaccharides, oligonucleotides, polynucleotides and hydrophobic, hydrophilic or amphiphilic organic nonprotein molecules;
• and/or nutrients;
• and/or cosmetic or plant-protection products.

25. Process for the preparation of a polyamino acid as defined in one of claims 1 to 14,
**characterized in that** said polyamino acid is obtained by polymerization of N-carboxyamino acid anhydrides (NCA), the polymer thus obtained being then hydrolyzed under suitable conditions to give the polymer in its acid form, said process further comprising coupling with an alpha-tocopherol.

26. Process according to claim 25,
**characterized in that** the coupling with an alpha-tocopherol consists in a coupling of alpha-tocopherol with an acid function of the polyamino acid, said coupling being performed by reacting, in a suitable solvent such as dimethylformamide (DMF), N-methylpyrrolidone (NMP) or dimethyl sulfoxide (DMSO), the polyamino acid with vitamin E in the presence of a carbodiimide as coupling agent.

27. Process for the preparation of a polyamino acid as defined in one of claims 1 to 14, comprising the synthesis of said polyamino acid by polymerization of an NCA derivative of alpha-tocopherol, which consists in:
1) synthesizing an NCA derivative of alpha-tocopherol-glutamate by coupling of an alpha-tocopherol with an acid function of a glutamate N-carboxyanhydride,
2) copolymerizing said NCA derivative of alpha-tocopherol-glutamate with a benzylglutamate NCA,
3) performing a selective hydrolysis reaction of the benzyl functions of the copolymer, in a mixture of trifluoroacetic acid and hydrobromic acid at room temperature.

28. Process according to claim 25,
**characterized in that** the coupling with an alpha-tocopherol is performed via a spacer consisting of 1 to 4 amino acids, said coupling being performed in the presence of a coupling agent such as a dialkylcarbodiimide and consisting in:
1) successive reactions of vitamin E with protected amino acids, which are then deprotected to have a graftable amine function on the polymer, or
1') a reaction of vitamin E with an oligopeptide,
2) coupling of the alpha-tocopherol-spacer bearing an amine function, with the carboxylic acid function of a polymer to form an amide bond.

29. Process for the preparation of a composition as defined in one of claims 16 to 24,
**characterized in that** the active principle comes into contact with said polyamino acids as defined in one of claims 1 to 14.

30. Process for the preparation:
• of medicinal products, in particular for oral, nasal, vaginal, ocular, subcutaneous, intravenous, intramuscular, intradermal, intraperitoneal or intracerebral administration, the active principles of these medicinal products possibly being, especially, proteins, glycoproteins, proteins linked to one or more polyalkylene glycol chains {for example polyethylene glycol (PEG), in which case they are referred to as "PEGylated" proteins}, peptides, polysaccharides, lipopolysaccharides, oligonucleotides, polynucleotides and hydrophobic, hydrophilic or amphiphilic organic nonprotein molecules;
• and/or nutrients;
• and/or cosmetic or plant-protection products;
**characterized in that** it consists essentially in using at least one polyamino acid as claimed in any one of claims 1 to 14 and/or the composition as claimed in any one of claims 15 to 24.
